# EUROPÄISCHE PATENTSCHRIFT

(11) **EP 0 743 316 B1**
(45) Veröffentlichungstag und Bekanntmachung des Hinweises auf die Patenterteilung: **06.11.2002**
(21) Anmeldenummer: 96107261.8
(22) Anmeldetag: 08.05.1996
(51) Int. Cl.: C07F 9/50, C07F 15/00, C07C 45/50, C07B 53/00

(54) **Optisch aktive Phosphine, deren Herstellung, deren Metallkomplexe und Anwendung in der asymmetrischen Synthese**
Optically active phosphines, their preparation, their metal complexes and their use in asymmetric synthesis
Phosphines optiquement actives, leur préparation, leurs complexes métalliques et leur utilisation en synthèse asymmétrique

(30) Priorität: 12.05.1995 DE 19516968
(43) Veröffentlichungstag der Anmeldung: 20.11.1996
(73) Patentinhaber: BASF Aktiengesellschaft, 67056 Ludwigshafen (DE)
(72) Erfinder: Stürmer, Rainer, Dr., 67127 Roedersheim (DE); Laupichler, Lothar, Dr., 69121 Heidelberg (DE); Knochel, Paul, Prof. Dr., 35037 Marburg (DE); Langer, Falk, 35037 Marburg (DE)

(56) Entgegenhaltungen:
- BRUNNER H ET AL: "ASYMMETRISCHE KATALYSEN, 88 1 KINETIK DER RH-KATALYSIERTEN HYDRIERUNG VON (Z)-(ALPHA)-N-ACETYLAMINOSUMTSAEURE MIT OPTISCH AKTIVEN SCHALENPHOSPHINEN ALS LIGANDEN ASYMMETRIC CATALYSIS, 88 1 KINETICS OF THE RH CATALYSED HYDROGENATION OF (Z)-(ALPHA)-N- ACETAMIDOCINNAMIC ACID WITH OPTICALLY ACTIVE LAYER-" ZEITSCHRIFT FUR NATURFORSCHUNG, TEIL B: ANORGANISCHE CHEMIE, ORGANISCHE CHEMIE, Bd. 49, Nr. 9, September 1994, Seiten 1305-1307, XP000466909
- "A CATALYZED ASYMMETRIC SYNTHESIS" ANGEWANDTE CHEMIE. INTERNATIONAL EDITION, Bd. 11, Nr. 11, November 1972, Seite 1023/1024 XP000616646
- LANGER F ET AL: "A new efficient preparation of polyfunctional phosphines using zinc organometallics" TETRAHEDRON LETT. (TELEAY,00404039);95; VOL.36 (26); PP.4591-4, PHILIPPS UNIV.;FACHBEREICH CHEMIE; MARBURG; D-35032; GERMANY (DE), XP002057218
- LONGEAU A ET AL: "Preparation and reactions of functionalized chlorodiorganophosphine-borane complexes using organozinc reagents" TETRAHEDRON LETT. (TELEAY,00404039);96; VOL.37 (13); PP.2209-12, FACHBEREICH CHEM. PHILIPPS-UNIV. MARBURG;MARBURG; D-35032; GERMANY (DE), XP002057219

## Beschreibung

Die vorliegende Erfindung betrifft optisch aktive Phosphine ein Verfahren zur Herstellung optisch aktiver Phosphine und deren Metallkomplexe sowie die Anwendung der Metallkomplexe für die enantioselektive Hydrierung, Hydroformylierung und Isomerisierung.

Für die Synthese optisch aktiver Verbindungen spielt die enantioselektive Hydrierung und Isomerisierung mit Rhodium und Rutheniumkomplexen eine große Rolle (z.B.Tani et.al. J. Am. Chem Soc 106, 5211, 1984; R. Noyori, Acc. Chem. Res. 23, 345 (1990) Die dabei zur Anwendung kommenden Katalysatoren, die meist aus einem optisch aktiven Diphosphinliganden und einer Rhodium- oder Rutheniumverbindung hergestellt werden, sind sehr teuer und nur durch aufwendige Herstellverfahren zugänglich.

Die bekannten Herstellmethoden für optisch aktive Phosphine sind durchweg vielstufig und beinhalten meist eine technisch aufwendige und teure Racematspaltung (z.B. EP-A 614901; EP-A 271311; H.B.Kagan, "Chiral Ligands for Asymmetric Catalysis" in Asymmetric Synthesis, Vol. 5(1985), Seite 13-23., EP-A 151282; EP-A 185882; R. Noyori, Acc. Chem. Res. 23, 345 (1990); EP-A 269395. Daher ist die industrielle Nutzung solcher Katalysatoren meist unwirtschaftlich.

In "A catalyzed asymmetric synthesis", Angew. Chemie, Int. Ed. Bd. 11, Nr. 11, Nov. 1972, Seite 1023/1024 werden asymmetrische Oligomerisierungen von Olefinen mittels π-Allylnickelhalogenid-Lewis-Säure Systemen beschrieben. Als Liganden werden u.a. Trismyrtanylphosphine verwendet.

Es bestand daher die Aufgabe, optisch aktive Phosphine bereitzustellen, die sich als Liganden für Übergangsmetallkomplexkatalysatoren eignen, bei deren Herstellung die oben angegebenen Nachteile vermieden werden.

Gefunden wurde ein Verfahren zur Herstellung von optisch aktiven Phosphinen, das dadurch gekennzeichnet ist, daß
a) ein optisch aktives Olefin in bekannter Weise hydroboriert wird,
b) das Reaktionsprodukt von Schritt a) mit einer Dialkylzinkverbindung oder einem Alkylzinkhalogenid umgesetzt wird,
c) das Reaktionsprodukt von Schritt b) mit einem Phophorhalogenid zum Phosphin umgesetzt wird.

Die Hydroborierung von Olefinen gemäß Schritt a) ist bekannt (z.B. Cragg, Organoboranes in Organic Synthesis, Marcel Dekker, NY, 1973).

Bevorzugt werden in Schritt a) Terpene, insbesondere die optisch aktiven Verbindungen (+)-alpha-Pinen, (-)-alpha-Pinen, beta-(+)-Pinen und beta-(-)-Pinen, (+)-delta2-Caren, (+)-delta3-Caren, (+)-Menthen, (+) und (-)-Camphen, (+)-Fenchen, (+) und (-)-Limonen, (+)-Menthadien, (+)-Longifolen mit Boran in Form seines Dimethylsulfid-, Tetrahydrofuran-, Ether- oder Amin-Adduktes umgesetzt um das Trialkyl,Dialkyl oder das Monoalkylboran zu erhalten.

Das Reaktionsprodukt von Schritt a) wird anschließend mit einer Dialkylzinkverbindung, bevorzugt Dimethyl-, Diethyl- oder Din-octylzink, umgesetzt um die entsprechende zinkorganische Verbindung mit den o.g. Terpenresten zu erhalten (Schritt b)).

Das Reaktionsprodukt von Schritt b) wird mit einem Phosphin, welches eine oder mehrere Fluchtgruppen trägt, umgesetzt. Als Fluchtgruppen eignet sich besonders Halogenreste, insbesondere Chlorreste.

Besonders bevorzugte Phosphine dieser Art sind das Phenylphosphordichlorid, Diphenylphosphorchlorid, Phosphortrichlorid, Phosphortribromid, Phosphortriiodid, 1,2-bis-(dichlorphosphino)-ethan, 1,3-bis-(dichlorphosphino)-propan und das 1,4-bis-(dichlorphosphino)-butan.

Nach Zugabe von Boran in Form seines Dimethylsulfid-, Tetrahydrofuran-, Ether- oder Aminadduktes wird das gesuchte Phosphin in Form seines Boran-Adduktes isoliert. Aus diesem Addukt kann das jeweilige Phosphin durch Erhitzen mit einem tiefsiedenden Amin, bevorzugt mit Ethylamin, Dimethylamin, Ammoniak, Methylamin, Trimethylamin, Triethylamin, Propylamin und besonders bevorzugt Diethylamin freigesetzt werden.

Weiterhin sind Gegenstand der Erfindung optisch aktive Phosphine der allgemeinen Formel II, 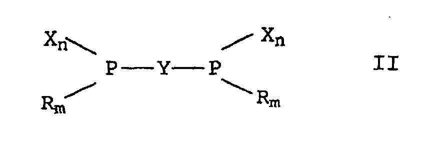 in der die Variablen folgende Bedeutung haben:
- X: ein optisch aktiver Terpenrest
- R: ein gegebenenfalls substituierter Phenylrest
- Y: ein Brückenglied mit 1-10 C-Atomen
- n: 1,2
- m: 0,1 mit der Maßgabe, daß n+m=2

Das Brückenglied Y kann aus einer unverzweigten oder verzweigten Alkyl- oder Arylalkylkette bestehen. Bevorzugt sind als Y unverzweigte Alkylketten mit 1-6 C-Atomen, besonders bevorzugt Ethan-, Propan und Butanreste.

Aus diesen neuen Phosphinen können dann in bekannter Weise (z.B. Uson, Inorg. Chim. Acta 73, 275 (1983, ,EP-A 0158875, EP-A 437690) durch Umsetzung mit Rhodium, Iridium oder Rutheniumkomplexen, die labile Liganden enthalten (z.B. [RuCl₂(COD)]n, Rh(COD)₂BF₄ , Rh(COD)₂ClO₄, [Ir(COD)Cl]₂, p-Cymol-Rutheniumchlorid-dimer) katalytisch aktive Komplexe synthestisiert werden.

Gegenstand der Erfindung sind daher auch Übergangsmetallkomplexe der allgemeinen Formel III oder IV 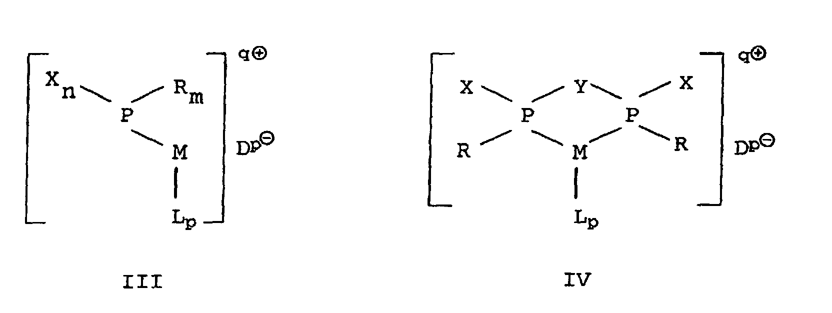 in der die Variablen X, Y, R, m, n die oben genannte Bedeutung und die übrigen Variablen die folgende Bedeutung haben:
- D: das Äquivalent eines nicht koordinierten Anions,
- L: einen organischen Liganden
- M: ein Übergangsmetall
- n: 1, 2, 3
- m: 0, 1, 2 mit der Maßgabe, dass n + m = 3
- p: 0 bis 4
- q: 0 bis 4.
- M: ist bevorzugt ein Metall aus der Gruppe Ru, Rh, und Ir.

Außerdem ist Gegenstand der Erfindung ein Verfahren zur Herstellung der oben genannten Übergangsmetallkomplexe, das dadurch gekennzeichnet ist, daß man eine Verbindung I oder II in an sich bekannter Weise mit einer Übergangsmetallverbindung V 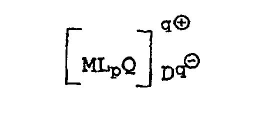 in der Q für eine Abgangsgruppe steht, umsetzt.

Auch die Verwendung der Übergangsmetallkomplexe III oder IV als Katalysatoren für asymmetrische Hydrierungsreaktionen, für asymmetrische Hydroformylierungsreaktionen und zur asymmetrischen Isomerisierung von Allylaminen zu Enaminen ist Gegenstand der Erfindung.

### Beispiel 1:

### Herstellung von (-) -1,2-bis(cis-dimyrtanylphosphino)-ethan

### Herstellung von Tri-cis-myrtanylboran

13,6 g (100 mmol) (-) - (1S) -beta-Pinen wurden auf -10°C gekühlt und innerhalb von 10 min mit 2,28 g (30 mmol) Boran-Dimethylsulfid-Addukt versetzt. Nach 10 min wurde die Suspension mit 15 ml Ether verdünnt und 2 h bei 0°C und 8 h bei Raumtemperatur nachgerührt. Im Vakuum wurden alle flüchtigen Bestandteile abgezogen. Es wurden 12,6 g ,99 % Ausbeute Tri-cis-myrtanylboran als weisser Feststoff isoliert.
1H-NMR (200 MHz, CDCl3) d= 0.81 (d, J= 9.4 Hz, 3H), 0.98 (s, 9H), 1.09 (s, 9H), 1.32 (m, 9H), 1.55 (m, 3H), 1.80 (m, 12H), 2.15-2.45 (m, 6H)

### Herstellung von Di-cis-myrtanylzink

12,5 g (29,6 mmol) Tri-cis- myrtanylboran wurden in 12 ml Hexan suspendiert und auf 0°C gekühlt. Nach Zugabe von 6,0 ml (60 mmol) Diethylzink wird 30 min bei 0°C gerührt. Das Hexan und überschüssiges Diethylzink wurden durch vierstündiges Rühren bei 40°C im Vakuum entfernt. Es wurden 15.0 g ( 44.1 mmol) Di-cis-myrtanylzink (99% Ausbeute) als farbloses Öl erhalten.
1H-NMR (200 MHz, CDCl3) d= 0.61 (dd, J= 7.5 und 9.0 Hz, 4 H), 0.86 (d, J= 9.2 Hz, 2H), 1.10 (s, 6H), 1.21 (s, 6H), 1.34 -1.53 (m, 2H), 1.70- 2.19 (m, 10 H), 2.30 (m, 4H)

### Herstellung von (-)-1,2-Bis(cis-dimyrtanylphosphino)-ethan-bisboran-Komplex

Zu einer Lösung von 6,61 g (6.0 mmol) Di-cis-myrtanylzink in 10 ml THF wurden bei 0°C 0.46 g (2 mmol) 1,2-bis-(dichlorphosphino)-ethan zugetropft. Nach beendigter Zugabe wurde das Kühlbad entfernt und 12 h auf 40°C erhitzt. Das Reaktionsgemisch wurde erneut auf 0°C gekühlt und mit 6 ml (6 mmol) einer 1M- Lösung von Boran-Dimethylsulfid-Komplex in Methylenchlorid versetzt. Nach 10 min bei 0°C wurde das Lösungsmittel entfernt und der Rückstand flashchromatographiert. Es werden 0,93 g des Phosphin-Boran-Komplexes als weisser Feststoff isoliert. (Ausbeute: 70 %)
Schmelzpunkt: 148 oC, Drehwert : -38.2° (c= 3.35 in Chloroform).
1H-NMR (200 MHz, CDCl3) d= 0.9 (d, J= 10.0 Hz, 4H), 0.99 (s, 12 H), 1.16 (s, 12 H), 1.35-1.55 (m, 4H), 1.60-1.96 (m, 28H), 1.96-2.20 (m, 4H), 2.20-2.40 (m, 8H)

### Herstellung von (-)-1,2 -bis(cis -dimyrtanylphosphino) -ethan

0,33 g (0,5 mmol) (-)-1,2-bis (cis-dimyrtanylphosphino)-ethan-bis-boran-Komplex wurde nach Zugabe von 1 ml (10 mmol) Diethylamin 45 min auf 50°C erhitzt. Dann wurde alles flüchtige Material bei 0,1 Torr entfernt und der Vorgang noch zweimal wiederholt. Es wurden 0,31 g (98 % Ausbeute) (-)-1,2-bis(cis-dimyrtanylphosphino)-ethan als weisser Feststoff vom Schmelzpunkt 120°C erhalten.
Drehwert: -24.7° (c= 3.0 in Chloroform)
13C-NMR (50 MHz, CDCl3) d= 17.3 (d, J= 30 Hz), 23.2, 24.2, 26.0, 27.7 (d, J= 1.2 Hz), 32.7 (dd, J= 10.7 und 30.9 Hz), 32.8, 35.7 (d, J= 2.1 Hz), 38.2, 40.6, 48.0 (q, J= 4.0 Hz)

### Beispiel 2

### Herstellung von (-)-1,4-Bis(cis-dimyrtanylphosphino)-butan-bisboran-Komplex

In analoger Weise wie in Beispiel 1 wurde der (-)-1,4-Bis(cis-dimyrtanylphosphino)-butan-bisboran- Komplex hergestellt.

Zu einer Lösung von 6,61 g (6.0 mmol) Di-cis-myrtanylzink in 10 ml THF wurden bei 0°C 0.52 g (2 mmol) 1,4-bis-(dichlorphosphino)-butan zugetropft. Nach beendigter Zugabe wurde das Kühlbad entfernt und 12 h auf 40°C erhitzt. Das Reaktionsgemisch wurde erneut auf 0°C gekühlt und mit 6 ml (6 mmol) einer 1M-Lösung von Boran-Dimethylsulfid-Komplex in Methylenchlorid versetzt. Nach 10 min bei 0°C wurde das Lösungsmittel entfernt und der Rückstand flashchromatographiert. Es werden 1,02 g des Phosphin-Boran-Komplexes als weisser Feststoff isoliert.
Drehwert : -40,2° (c= 3,00 in Chloroform). 1H-NMR (200 MHz, CDCl3) d= 0.95 (d, J= 10,2 Hz, 4H), 1,02 (s, 12 H), 1,16 (s, 12H), 1,30-1,65 (m, 8H), 1,60-1,96 (m, 28H), 1,96-2,10 (m, 4H), 2,20-2,30 (m, 8H)

Die Dekomplexierung zum freien Phosphin wurde durch mehrmalige Zugabe von Diethylamin und Abdestillieren der flüchtigen Bestandteile erreicht.

### Beispiel 3

### Herstellung von Tris-cis-myrtanylphosphin

In analoger Weise konnte z.B tris-cis-myrtanylphosphin aus beta-Pinen und Phosphortrichlorid hergestellt werden. Der Borankomplex dieses Phosphins zeigt im 1H-NMR-Spektrum ( 200 MHz, CDCl3) d= 0.88 ( d, J= 9.8 Hz, 3H), 0.96 (s, 9 H), 1.13 (s, 9H), 1.43 (m, 3H), 1.66 (m, 8H); 1.84 (m, 12H), 2.10 (m, 3H), 2.23 (m, 6H).

### Beispiel 4

### Anwendungen der Phosphine für Hydrierung

In einem 200 ml Laborautoklaven mit Glaseinsatz der Fa. Roth wurden 3,0 mg p-Cymol-Rutheniumchlorid-dimer (Aldrich) in 20 ml Methanol vorgelegt und mit 6,4 mg (-)-1,2-bis(cis-dimyrtanylphosphino) -ethan versetzt. Nach 15 min Rühren bei Raumtemperatur wurde zu der so bereiteten gelben Katalysatorlösung 260 mg (2.0 mmol) Acetessigsäureethylester gegeben.

Der Autoklav wurde verschlossen und es wurde bei einem Wasserstoffdruck von 100 bar und einer Temperatur von 50°C 12 h hydriert. Nach Entspannen des Autoklaven wurde das Reaktionsgemisch vom Lösungsmittel befreit und der Rückstand kugelrohrdestilliert. Es wurden 220 mg (R)-3-Hydroxybutansäure-ethylester mit einem Drehwert von -34° (c= 1, Chloroform) erhalten. Das entspricht einem Enantiomerenüberschuß von 74 %.

### Beispiel 5

Anwendung der Phosphine bei der Hydrierung In einem 200 ml Laborautoklaven mit Glaseinsatz der Fa. Roth wurden 3,0 mg p-Cymol-Rutheniumchlorid-dimer (Aldrich) in 20 ml Methanol vorgelegt und mit 6,4 mg (-)-1,2-bis(cis-dimyrtanylphosphino)-ethan versetzt. Nach 15 min Rühren bei RT wurde zu der so bereiteten gelben Katalysatorlösung 296 mg (2,0 mmol)
trans-2-Methyl-3-phenyl-2-propen-1-ol gegeben. Der Autoklav wurde verschlossen und es wurde bei einem Wasserstoffdruck von 100 bar und einer Temperatur von 50°C 12 h hydriert. Nach Entspannen des Autoklaven wurde das Reaktionsgemisch vom Lösungsmittel befreit und der Rückstand kugelrohrdestilliert.

Es wurden 230 mg 2-Methyl-3-Phenyl-2-propan-1-ol erhalten. Der ee-Wert beträgt 76 % ((R)-(-)-(9-Anthryl),2,2,2-trifluor-ethanol als NMR-Shiftreagens)

### Beispiel 6

### Anwendung der Phosphine bei der Hydroformylierung

In einem 200 ml Laborautoklaven mit Glaseinsatz der Fa. Roth wurden 3,90 mg Rhodium-biscyclooctadien-tetrafluoroborat in 20 ml Benzol vorgelegt und mit 6,4 mg (-)-1,2-bis(cis-dimyrtanylphosphino)-ethan versetzt. Nach 15 min Rühren bei RT wurde zu der so bereiteten gelben Katalysatorlösung 236 mg (2,0 mmol) alpha-Methylstyrol gegeben. Nach Aufpressen von 100 bar eines 1:1 Gemisches aus Wasserstoff und Kohlenmonoxid wurde 24 h bei 50°C gerührt. Nach Öffnen des Autoklaven wurde das Reaktionsgemische über 2x2 cm Aluminiumoxid filtriert und das Filtrat vom Lösungsmittel befreit. Erhalten wurden 200 mg eines Gemisches von 3-Phenyl-butan-1-al und 2-Phenyl-2-Methylpropan-1-al im Verhältnis 80 : 20 (1-H-NMR). Der Enantiomerenüberschuß des 3-Phenyl-butan-1-als beträgt 65%, bestimmt durch Messung des NMR-Shifts nachReduktion zum entsprechenden Alkohol mit NaBH4.

### Beispiel 7

### Anwendung der Phosphine bei der Isomerisierung von Allylaminen.

In einem 200 ml Laborautoklaven mit Glaseinsatz der Fa. Roth wurden 3,90 mg Rhodium-biscyclooctadien-tetrafluoroborat in 20 ml THF vorgelegt und mit 6,4 mg (-)-1,2-bis(cis-dimyrtanylphosphino)-ethan versetzt. Nach 15 min Rühren bei RT wurde zu der so bereiteten gelben Katalysatorlösung 306 mg (2,0 mmol) Geranylamin (Aldrich) gegeben. Nach Aufpressen von 2 bar Argon wurde 24 h bei 50°C gerührt. Nach Öffnen des Autoklaven wurde das Reaktionsgemische über 2x2 cm Aluminiumoxid filtriert und das Filtrat vom Lösungsmittel befreit. Das Rohprodukt wurde in 10 ml 30 % Essigsäure in Wasser aufgenommen, 30 min bei RT gerührt, dann dreimal mit je 30 ml n-Hexan extrahiert. Die vereinigten organische Phasen wurden über Natriumsulfat getrocknet und im Vakuum vom Lösungsmittel befreit.

Es werden 270 mg Citronellal als farbloses Öl erhalten. Der Enantiomerenüberschuß liegt bei 72 % , Drehwert :-12.5° (neat)

## Patentansprüche

1. Optisch aktive Phosphine der allgemeinen Formel II, 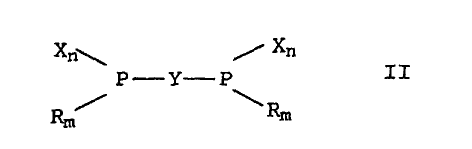 in der die Variablen folgende Bedeutung haben:
X ein optisch aktiver Terpenrest
R ein gegebenenfalls substituierter Phenylrest
Y ein Brückenglied mit 1-10 C-Atomen
n 1,2
m 0,1 mit der Maßgabe, daß n+m=2

2. Phosphine nach Anspruch 1, **dadurch gekennzeichnet daß** X einen Rest aus der Gruppe, die von (+)-alpha-Pinen, (-)-alpha-Pinen, beta-(+)-Pinen, beta-(-)-Pinen, (+)-delta2-Caren, (+)-delta3-Caren, (+)-Menthen, (+) und (-)-Camphen, (+)-Fenchen, (+) und (-)-Limonen, (+)-Menthadien, (+)-Longifolen gebildet wird, bedeutet.

3. Verfahren zur Herstellung von optisch aktiven Phosphinen, **dadurch gekennzeichnet, daß**
a) ein optisch aktives Olefin in bekannter Weise hydroboriert wird,
b) das Reaktionsprodukt von Schritt a) mit einer Dialkylzinkverbindung umgesetzt wird,
c) das Reaktionsprodukt von Schritt b) mit einem Phophorhalogenid zum Phosphin umgesetzt wird.

4. Verfahren nach Anspruch 3, **dadurch gekennzeichnet, daß** als optisch aktives Olefin in Schritt a) ein Vertreter der Gruppe aus (+)-alpha-Pinen, (-)-alpha-Pinen, beta-(+)-Pinen, beta-(-)-Pinen, (+)-delta2-Caren, (+)-delta3-Caren, (+)-Menthen, (+) und (-)-Camphen, (+)-Fenchen, (+) und (-)-Limonen, (+)-Menthadien, (+)-Longifolen verwendet wird.

5. Verwendung der Phosphine gemäß Anspruch 1 und 2 zur Komplexierung von Übergangsmetallverbindungen.

6. Übergangsmetallkomplexe der allgemeinen Formel III oder IV 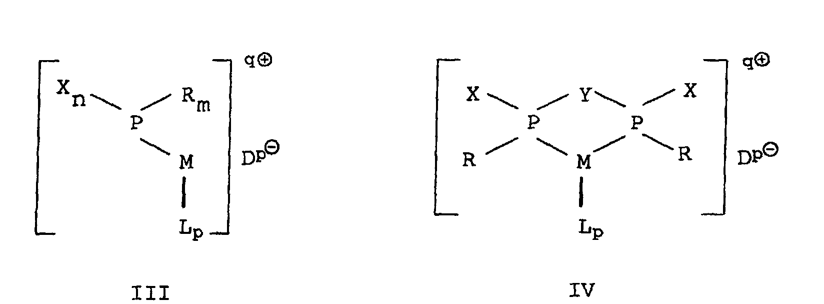 in der die Variablen folgende Bedeutung haben:
X ein optisch aktiver Terpenrest
R ein gegebenenfalls substituierter Phenylrest
Y ein Brückenglied mit 1-10 C-Atomen in der Formel I
D das Äquivalent eines nicht koordinierten Anions,
L einen organischen Liganden
M ein Übergangsmetall aus der Gruppe Ru, Rh und Ir,
n 1, 2, 3
m 0, 1, 2 mit der Maßgabe, dass n + m = 3
p 0 bis 4
q 0 bis 4.

7. Verfahren zur Herstellung der Übergangsmetallkomplexe gemäß Anspruch 6, **dadurch gekennzeichnet, daß** man eine Verbindung I oder II
XₙPRₘ I 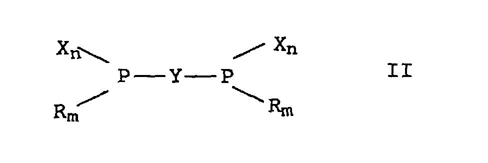
in der die Variablen folgende Bedeutung haben:
X ein optisch aktiver Terpenrest
R ein gegebenenfalls substituierter Phenylrest
Y ein Brückenglied mit 1-10 C-Atomen in der Formel I
n 1,2,3
m 0,1,2 mit der Maßgabe, daß n+m=3 in der Formel II
n 1,2
m 0,1 mit der Maßgabe, daß n+m=2
in an sich bekannter Weise mit einer Übergangsmetallverbindung V 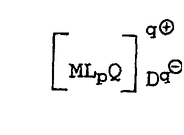 in der Q für eine Abgangsgruppe steht, umsetzt.

8. Verwendung der Übergangsmetallkomplexe III oder IV als Katalysatoren für asymmetrische Hydrierungsreaktionen.

9. Verwendung der Übergangsmetallkomplexe III oder IV als Katalysatoren für asymmetrische Hydroformylierungsreaktionen.

10. Verwendung der Übergangsmetallkomplexe III oder IV als Katalysatoren zur asymmetrischen Isomerisierung von Allylaminen zu Enaminen.

## Claims

1. An optically active phosphine of the general formula II 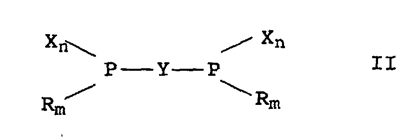 where the variables have the following meanings:
X is an optically active terpene radical,
R is an unsubstituted or substituted phenyl radical,
Y is a bridge member having 1-10 C atoms
n is 1 or 2 and
m is 0 or 1 with the proviso that n+m = 2.

2. A phosphine as claimed in claim 1, wherein X is a radical from the group which is formed from (+)-alpha-pinene, (-)-alpha-pinene, (+)-beta-pinene, (-)-beta-pinene, (+)-delta2-carene, (+)-delta3-carene, (+)-menthene, (+)- and (-)-camphene, (+)-fenchene, (+)- and (-)-limonene, (+)-menthadiene and (+)-longifolene.

3. A process for preparing optically active phosphines, which comprises
a) hydroborating an optically active olefin in a known manner,
b) reacting the reaction product from step a) with a dialkylzinc compound,
c) reacting the reaction product from step b) with a phosphorus halide to give the phosphine.

4. A process as claimed in claim 3, wherein the optically active olefin used in step a) is a representative of the group consisting of (+)-alpha-pinene, (-)-alpha-pinene, (+)-beta-pinene, (-)-beta-pinene, (+)-delta2-carene, (+)-delta3-carene, (+)-menthene, (+)- and (-)-camphene, (+)-fenchene, (+)- and (-)-limonene, (+)-menthadiene and (+)-longifolene.

5. The use of the phosphines as claimed in claims 1 and 2 for complexing transition metal compounds.

6. A transition metal complex of the general formula III or IV 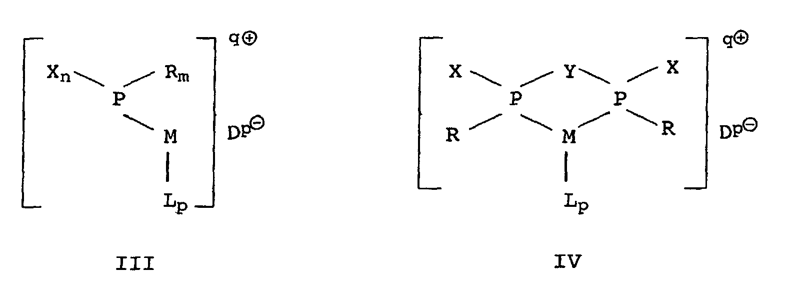 where the variables have the following meanings:
X is an optically active terpene radical,
R is an unsubstituted or substituted phenyl radical,
Y is a bridge member having 1-10 C atoms
D is the equivalent of a noncoordinated anion,
L is an organic ligand,
M is a transition metal from the group consisting of Ru, Rh and Ir,
n is 1, 2 or 3
m is 0,1 or 2 with the proviso that n+m = 3
p is 0 to 4 and
q is 0 to 4.

7. A process for preparing the transition metal complexes as claimed in claim 6, which comprises reacting a compound I or II
XₙPRₘ I 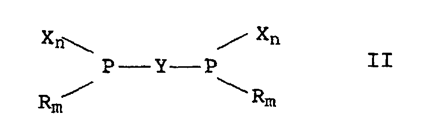
where the variables have the following meanings:
X is an optically active terpene radical,
R is an unsubstituted or substituted phenyl radical,
Y is a bridge member having 1-10 C atoms in the formula I
n is 1,2 or 3 and
m is 0,1 or 2 with the proviso that n+m = 3 in the formula II
n is 1 or 2 and
m is 0 or 1 with the proviso that n+m = 2.
in a manner known per se with a transition metal compound V 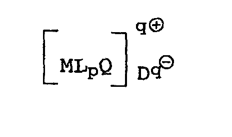 where Q is a leaving group.

8. The use of the transition metal complexes III or IV as catalysts for asymmetric hydrogenation reactions.

9. The use of the transition metal complexes III or IV as catalysts for asymmetric hydroformylation reactions.

10. The use of the transition metal complexes III or IV as catalysts for the asymmetric isomerization of allylamines to enamines.

## Revendications

1. Phosphines possédant l'activité optique et répondant à la formule générale II 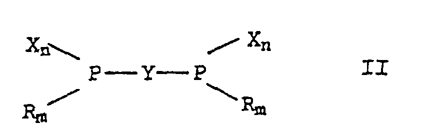 dans laquelle les symboles ont les significations suivantes :
X représente un radical de terpène possédant l'activité optique,
R représente un groupe phényle éventuellement substitué,
Y représente un pont en C1-C10,
n est égal à 1 ou 2,
m est égal à 0 ou 1, sous réserve que n+m=2.

2. Phosphines selon la revendication 1, **caractérisées par le fait que** X représente un radical du groupe formé par le (+)-alpha-pinène, le (-)-alpha-pinène, le bêta-(+)-pinène, le bêta-(-)-pinène, le (+)-delta2-carène, le (+)-delta3-carène, le (+)-menthène, le (+) et le (-)-camphène, le (+)-fenchène, le (+) et le (-)-limonène, le (+)-menthadiène, le (+)-longifolène.

3. Procédé pour la préparation de phosphines possédant l'activité optique, **caractérisé par le fait que**
a) on soumet une oléfine possédant l'activité optique à hydroboration de manière connue,
b) on fait réagir le produit de réaction du stade a) avec un dérivé de dialkyl-zinc,
c) on convertit le produit de réaction du stade b), à l'aide d'un halogénure de phosphore, en la phosphine.

4. Procédé selon la revendication 3, **caractérisé par le fait que** l'on utilise en tant qu'oléfine possédant l'activité optique au stade a), un représentant du groupe consistant en le (+)-alpha-pinène, le (-)-alpha-pinène, le bêta-(+)-pinène, le bêta-(-)-pinène, le (+)-delta2-carène, le (+)-delta3-carène, le (+)-menthène, le (+) et le (-)-camphène, le (+)-fenchène, le (+)- et le (-)-limonène, le (+)-menthadiène, le (+)-longifolène.

5. Utilisation des phosphines selon les revendications 1 et 2 pour la complexation de dérivés de métaux de transition.

6. Complexes de métaux de transition de formule générale III ou IV 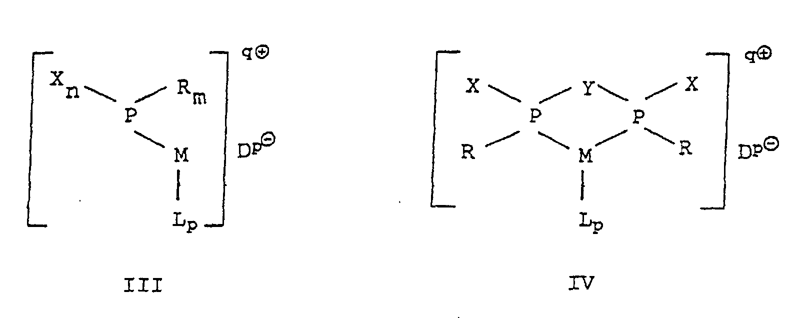 dans laquelle les symboles ont les significations suivantes :
X représente un radical de terpène possédant l'activité optique,
R représente un groupe phényle éventuellement substitué,
Y représente un pont en C1-C10 dans la formule I,
D représente l'équivalent d'un anion non coordinant,
L représente un ligand organique,
M représente un métal de transition du groupe consistant en Ru, Rh et Ir,
n est égal à 1,2 ou 3,
m est égal à 0, 1 ou 2 sous réserve que n + m = 3,
p va de 0 à 4,
q va de 0 à 4.

7. Procédé pour la préparation des complexes de métaux de transition selon la revendication 6, **caractérisé en ce que** l'on fait réagir de manière connue en soi un composé I ou II
XₙPRₘ I 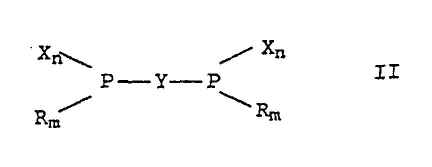
pour lesquels les symboles ont les significations suivantes
X représente un radical de terpène possédant l'activité optique,
R représente un groupe phényle éventuellement substitué,
Y représente un pont en C1-C10 dans la formule I,
n est égal à 1, 2 ou 3,
m est égal à 0, 1 ou 2 sous réserve que n + m = 3 dans la formule II
n est égal à 1 ou 2,
m est égal à 0 ou 1 sous réserve que n + m = 2, avec un dérivé de métal de transition V 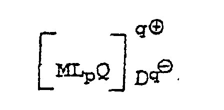 pour lequel Q représente un groupe éliminable.

8. Utilisation des complexes de métaux de transition III ou IV en tant que catalyseurs pour des réactions d'hydrogénation asymétrique.

9. Utilisation des complexes de métaux de transition III ou IV en tant que catalyseurs pour des réactions d'hydroformylation asymétrique.

10. Utilisation des complexes des métaux de transition III ou IV en tant que catalyseurs pour l'isomérisation asymétrique d'allylamines en énamines.
